# EUROPEAN PATENT SPECIFICATION

(11) **EP 1 339 371 B1**
(45) Date of publication and mention of the grant of the patent: **22.02.2012**
(21) Application number: 01943604.7
(22) Date of filing: 12.06.2001
(51) Int. Cl.: A61H 23/04, A61H 1/02, A61B 17/135

(54) **LEAKAGE DETECTION METHOD FOR A PRESSURISED MEDICAL APPLIANCE**
LECKPRÜFUNGSVERFAHREN FÜR EINE UNTER DRUCK STEHENDE MEDIZINISCHE EINRICHTUNG
PROCEDE DE DETECTION DE FUITE SUR UN APPAREIL MEDICAL SOUS PRESSION

(30) Priority: 17.06.2000 GB 0014789
(43) Date of publication of application: 03.09.2003
(73) Proprietor: Covidien AG, 8212 Neuhausen am Rheinfall (CH)
(72) Inventor: COOK, Gordon, Andover Hampshire SP11 9HX (GB)
(74) Representative: Allsop, John Rowland
(86) International application number: PCT/GB2001/002557
(87) International publication number: WO 2001/097747

(56) References cited:
- EP-A- 0 992 230
- WO-A-93/12708
- WO-A-96/06569
- GB-A- 2 270 472
- US-A- 5 935 146
- US-A- 5 951 502
- US-A- 6 051 016

## Description

### FIELD OF THE INVENTION

The present invention relates to a medical appliance employing a fluid inflatable bag or bladder for applying cyclical pressure pulses to various parts of the human body thereby to enhance the circulation of blood and particularly to a method for determining the existence of fluid leakage in the appliance prior to normal operation.

### BACKGROUND OF THE INVENTION

A medical appliance known as the A-V IMPULSE SYSTEM™ for applying a pumping pressure to various parts of the human body has gained wide popularity and acceptance in the medical field throughout the world in the treatment of certain medical conditions such as post-operative and post-traumatic pain and swelling and pre-operative and post-operative prophylaxis of deep vein thrombosis.

Its most common use is by application to the plantar arch of the human foot and to this end the device comprises an inflatable bladder, such as sold and marketed under the trade mark ImPad®, which in use is held in the plantar arch, the bladder being coupled to a control unit which is adapted to pump air under pressure to inflate and deflate the bladder cyclically thereby to apply a special pumping action to the plantar arch which assists in promoting venous blood flow return from the foot to the rest of the body.

The medical appliance as above described may be used and operated in a hospital environment under the supervision of trained staff or by patients themselves in a home environment.

If the hose connections between the bladder and the control unit of the A-V Impulse System™ are not fitted properly or if the bladder itself is defective due to the presence of holes or splits, then of course air will leak from the system and the full benefit of the therapeutic effects of the appliance will not be achieved.

Moreover incorrectly fitted hose connections between the control unit and the bladder cause the control unit at the beginning of a treatment process to emit pulses of air at high velocity which can startle a new, elderly or clinically unsupported patient especially when the device is being used in a home environment.

Consequently particularly in patient groups of this type acceptance of the product can be jeopardised and the benefits of treatment compromised.

For representative prior art in connection with the present invention reference may be had to US Patent 5,951,502 which discloses a gradient sequential compression system for preventing deep vein thrombosis, GB Patent 2 270 472 which relates to an apparatus for applying compression to the limbs of the human body and to International Patent Publication WO 96/06569 which pertains to physiologic inflatable tourniquet device for facilitating surgical procedures performed on upper and lower limbs of the human body. US-A-5 951 502 discloses a method according to the preamble of claim 1 and an appliance according to the preamble of claim 3.

### SUMMARY OF THE INVENTION

It is an object of the present invention to modify the appliance of the prior art so that normal operation is not commenced until system integrity has been achieved that is before extraneous air leakage in the system connecting parts which couple the control unit to the bladder, or from a defective bladder, are eliminated.

According to one aspect of the invention there is provided a detection method for detecting the presence of leakage in a pressurised medical appliance for use on a patient to enhance the circulation of blood in the body the method being carried out prior to normal operation of the appliance on the patient, the appliance including an air compressor and an inflatable bag or bladder coupled to the air compressor through the intermediary of a fill valve allowing air to pass to the bladder and a vent valve for venting the appliance, the fill valve and the vent valve being selectively operable during said normal operation to deliver cyclical pulses of air to inflate and deflate the bladder, characterised in the detection method comprises
a. opening the fill valve and vent valve to vent the appliance to atmospheric pressure,
b. closing the vent valve,
c. pressurising the appliance by means of the air compressor to a pre-determined threshold value,
d. monitoring the pressure gradient over a period of time to said threshold value and
e. comparing the pressure gradient over said period of time or at intervals of time within said period of time with a pre-specified pressure gradient indicative of system integrity with respect to appliance air leakage whereby to determine the presence or otherwise of air leakage in the appliance, said pre-specified pressure gradient being based upon achieving an appliance pressure of 10 mmHg within 3 seconds of said air compressor.

Advantageously the method of control is by means of a computer programmed to operate the air compressor and the fill and vent valves as required.

According to another aspect of the invention there is provided a medical inflation appliance for use on a patient to enhance the circulation of blood in the body comprising an air compressor and an inflatable bladder coupled to the air compressor, a fill valve for allowing air to pass to the bladder, a vent valve for venting the appliance, the fill and vent valves being selectively operable to deliver cyclical pulses of pressurised air from the compressor periodically to inflate the bladder when in normal operation on the patient, the appliance being characterised by computer means programmed for selective operation of the fill valve and the vent valve prior to said normal operation whereby to detect for air leakage in the appliance prior to said normal operation, the selective operation, comprising:
a. opening the fill valve and vent valves to vent the appliance to atmospheric pressure,
b. closing the vent valve,
c. pressurising the appliance by means of the air compressor to a pre-determined threshold value,
d. monitoring the pressure gradient over a period of time to said threshold value and
e. comparing the pressure gradient over said period of time or at intervals of time within said period of time with a pre-specified pressure gradient indicative of appliance integrity with respect to appliance air leakage whereby to determine the presence or otherwise of air leakage in the appliance said pre-specified pressure gradient being based upon achieving an appliance pressure of 10 mmHg within 3 seconds of said air compressor.

### BRIEF DESCRIPTION OF THE DRAWINGS

The invention will now be described by way of example with reference to the accompanying drawings wherein;
Figure 1 is a schematic diagram of a medical appliance comprising a pressurised air supply control unit coupled in use to two inflatable bladders for applying cyclical pressure to a part of the human body and illustrating the initial stage of performing the detection method according to the present invention;
Figure 2 is a schematic diagram identical to that shown in Figure 1 but illustrating the operation of the air supply control in accordance with the final stage of the detection method in accordance with the invention and
Figure 2A is a graph illustrating preferred detection parameters in accordance with the method of the invention.

### PREFERRED EMBODIMENTS OF THE INVENTION

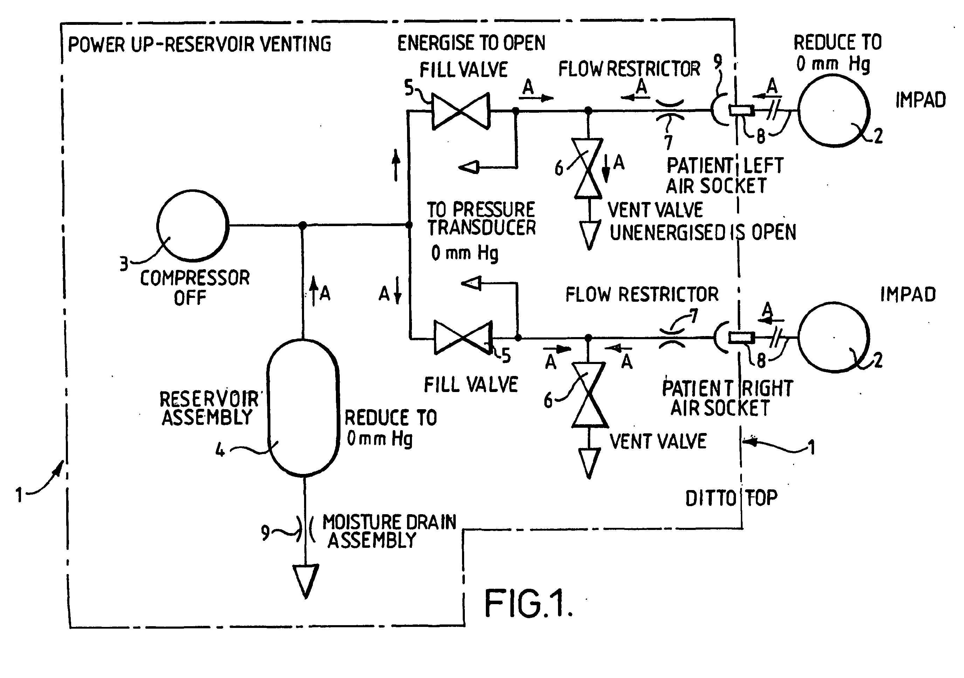

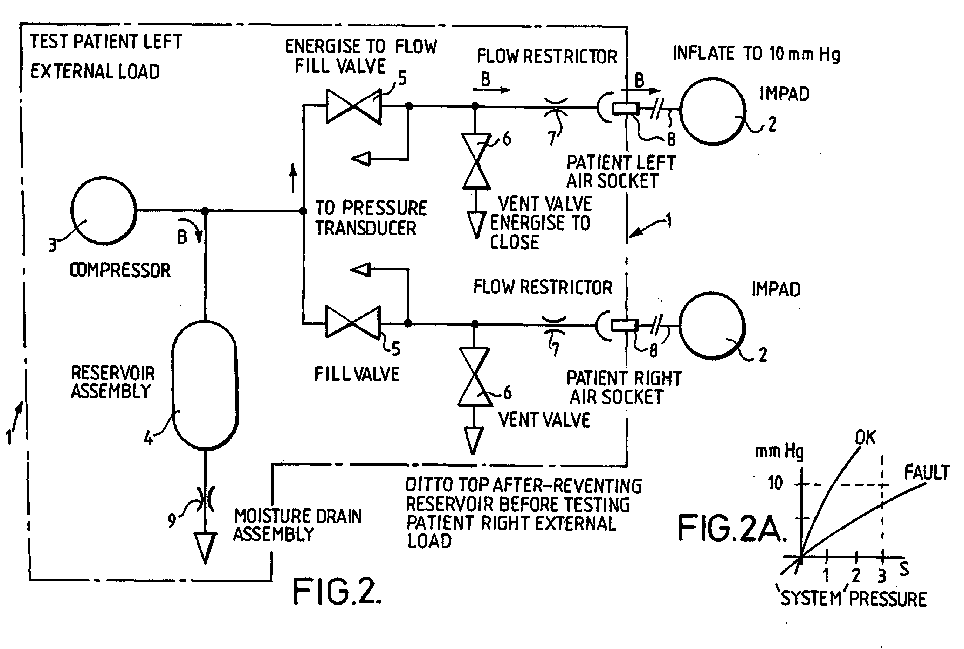

With reference to the drawings Figures 1 and 2 illustrate in schematic form the essential components of the medical appliance herein before referred to as the A-V IMPULSE SYSTEM™, for applying a pumping action to the body to enhance blood circulation through the intermediary of an inflatable bag or bladder.

The appliance comprises a control unit 1 coupled to at least one inflatable bag or bladder 2. In the illustration shown in the drawings the appliance is set up for use to inflate two bladders 2 for positioning respectively in the plantar arches of the feet of the human body and held in that position by a specially designed sling or foot slipper (not shown).

The control unit 1 comprises an air compressor 3, a storage reservoir 4 for compressed air and a fill valve 5 leading to each inflatable bladder 2 from the reservoir 4.

A vent valve 6 is positioned between each fill valve 5 and its associated bladder 2 with a flow restrictor 7 for controlling the flow of pressurised air immediately in advance of each inflatable bladder 2.

In use the patient, or attendant medical personnel, couples the control unit 1 to the bladders 2 when installed on the feet by hose connections 8 which fit into air sockets 9 provided on the housing (not shown) of the control unit 1, and the tube or connector (not shown) of the bladders 2.

In normal operation the compressor 3 is set to run continuously to charge the reservoir 4. Water condensate from compression of the air is discharged from the moisture drain assembly 9 in communication with the reservoir 4.

Air is not allowed to pass the fill valves 5 which remain normally closed. The bladders 2 are vented to atmospheric pressure through the vent valves 6 which are normally open.

The rapid impulse inflation pressure hold and venting are controlled by actuation of the fill and vent valves 5,6 during which the pressure in the reservoir 4 is fluctuating but always at greater than atmospheric pressure.

As referred to earlier in this disclosure unless the pressurised system is free of extraneous leaks, pulses of high velocity air flow from the air outlet sockets 9 or hose connection 8 of the control unit 1 which can cause distress to the patient using the appliance.

This problem has been dealt with in accordance with the present invention by an arrangement which does not allow the control unit 1 to operate at normal conditions while extraneous leaks are present in the system so that instead of charging the reservoir 4 to full operating pressure at the commencement of a medical treatment the system is first vented to atmosphere and then pressurised over a pre-determined period of time to a pre-determined threshold value to test for leakage and before pulses of pressurised air are delivered to the bladders 2.

Thus in this initial stage which may be termed an air leakage check stage, all residual pressure within the reservoir 4, internal tubing, fill and vent valves, 5,6 air hoses 8 or the inflatable bladder 2 is vented to atmospheric pressure by activating the fill valves 5 to open whilst leaving the vent valves 6 inactivated and therefore also in the open position, this taking place when the control unit 1 is turned on but before the air compressor 3 is started. This is illustrated by directional arrows A in Figure 1.

When the system has been sufficiently vented the vent valves 6 are activated to prevent air escaping from the system and with the fill valves 5 activated and therefore in the open position the compressor 3 is energised to raise the system pressure from atmospheric. This is illustrated by directional arrows B in Figure 2.

The system pressure is monitored as it rises from zero until threshold points are reached which are indicative or otherwise of system integrity.

In a preferred aspect of the method in accordance with the invention system integrity is considered to be achieved if a system pressure of 10mmHg is attained within three seconds from energisation of the compressor 3, see Figure 2A.

The pressure gradient may also be monitored and measured at various points whereby to determine the presence or otherwise of extraneous air leakage in the system.

The medical appliance shown in the drawings is a two channel system and with the method according to the invention both left and right sides may be checked sequentially for air leakage while ensuring that pressure is returned to atmospheric after testing one channel and before testing the other.

This does not preclude of course the possibility of testing both channels together but in practice this is less preferable.

Provided that the pressure limits are achieved within the permissible period the control unit 1 is allowed to continue to charge the reservoir 4 to normal working pressure by deactivating the fill valve 5 to close and deactivating the vent valve 6 to open and once normal conditions are attained impulse pressure is applied to the bladders 2.

Should the pressure limits not be achieved an alarm, not shown, is activated to warn the user who is then able to check the connections between the control unit and the bladder 2 and the integrity of the bladders 2.

As mentioned above a principal advantage of the method according to the invention is that should there be air leakage such will occur at relatively low pressure and at a steady rather than a pulsed state.

Apart from patient alarm which may be caused by pulses of leaking air at high velocity, the method according to the invention avoids unnecessary delay and promotes confidence in the set up of the device before actual treatment commences.

The normal detection method as described above is achieved without changing the basic structure of the appliance and may be controlled by a mini computer device installed in the appliance and programmed to operate the existing air valves 5,6 in an initial check stage which differs from the later selective operation of the air valves 5,6 during normal operation whereby to achieve pulsed air flow.

## Claims

1. A detection method for detecting the presence of leakage in a pressurised medical appliance (1) for use on a patient to enhance the circulation of blood in the body the method being carried out prior to normal operation of the appliance on the patient, the appliance including an air compressor (3) and an inflatable bag or bladder (2) coupled to the air compressor (3) through the intermediary of a fill valve (5) allowing air to pass to the bladder (2) and a vent valve (6) for venting the appliance, the fill valve (5) and the vent valve (6) being selectively operable during said normal operation to deliver cyclical pulses of air to inflate and deflate the bladder (2), **characterised in** the detection method comprises
a. opening the fill valve (5) and vent valve (6) to vent the appliance to atmospheric pressure,
b. closing the vent valve (5),
c. pressurising the appliance by means of the air compressor (3) to a pre-determined threshold value,
d. monitoring the pressure gradient over a period of time to said threshold value and
e. comparing the pressure gradient over said period of time or at intervals of time within said period of time with a pre-specified pressure gradient indicative of system integrity with respect to appliance air leakage whereby to determine the presence or otherwise of air leakage in the appliance, said pre-specified pressure gradient being based upon achieving an appliance pressure of 10 mmHg within 3 seconds of energisation of said air compressor (3).

2. A detection method as claimed in claim 1 wherein said steps (a) through (e) are performed by a computer programmed to operate the air compressor (3) and the fill valve (5) and vent valve (6).

3. A medical inflation appliance for use on a patient to enhance the circulation of blood in the body comprising an air compressor (3) and an inflatable bladder (2) coupled to the air compressor (3), a fill valve (5) for allowing air to pass to the bladder (2), a vent valve (6) for venting the appliance, the fill and vent valves (5,6) being selectively operable to deliver cyclical pulses of pressurised air from the compressor (3) periodically to inflate the bladder (2) when in normal operation on the patient, the appliance being **characterised by** computer means programmed for selective operation of the fill valve (5) and the vent valve (6) prior to said normal operation whereby to detect for air leakage in the appliance prior to said normal operation, the selective operation, comprising:
a. opening the fill valve (5) and vent valves (6) to vent the appliance to atmospheric pressure,
b. closing the vent valve (6),
c. pressurising the appliance by means of the air compressor (3) to a pre-determined threshold value,
d. monitoring the pressure gradient over a period of time to said threshold value and
e. comparing the pressure gradient over said period of time or at intervals of time within said period of time with a pre-specified pressure gradient indicative of appliance integrity with respect to appliance air leakage whereby to determine the presence or otherwise of air leakage in the appliance said pre-specified pressure gradient being based upon achieving an appliance pressure of 10 mmHg within 3 seconds of energisation of said air compressor (3).

## Patentansprüche

1. Leckprüfungsverfahren für eine mit Druck beaufschlagte medizinische Vorrichtung (1) zur Anwendung an einem Patienten für die Unterstützung der Blutzirkulation im Körper, wobei das Verfahren vor der bestimmungsgemäßen Anwendung der Vorrichtung an einem Patienten durchgeführt wird, und wobei die Vorrichtung einen Druckluftkompressor (3) und einen über das Zwischenstück eines Füllventils (5) und eines Entlüftungsventils (6) mit dem Druckluftkompressor gekoppelten aufblasbaren Sack oder Balg (2) aufweist, wobei das Füllventil den Lufteintritt in den Balg (2) ermöglicht und das Entlüftungsventil zur Entlüftung der Vorrichtung dient, wobei das Füllventil (5) und das Entlüftungsventil (6) während der bestimmungsgemäßen Anwendung selektiv bedienbar sind, um zyklische Luft-Pulse zum Aufblasen und Entleeren des Balges (2) bereitzustellen, **dadurch gekennzeichnet dass** das Prüfungsverfahren aufweist
a. Öffnen des Füllventils (5) und des Entlüftungsventils (6) zur Entlüftung der Vorrichtung auf atmosphärischen Druck,
b. Schließen des Entlüftungsventils (5),
c. Beaufschlagen der Vorrichtung mit Druck mittels des Druckluftkompressors (3) bis zu einem bestimmten Schwellenwert,
d. Überwachen des Druckgradienten zum Schwellenwert über einen Zeitraum,
e. Vergleichen des Druckgradienten über den Zeitraum oder zu Zeitintervallen innerhalb des Zeitraumes mit einem vorher festgelegten Druckgradienten, welcher die Systemintegrität bezogen auf die Undichtigkeit der Vorrichtung anzeigt, wobei zur Feststellung einer Undichtigkeit der Vorrichtung der vorher festgelegte Druckgradient auf dem Erreichen eines Druckes in der Vorrichtung von 10 mmHg innerhalb von 3 Sekunden nach Einschalten des Druckluftkompressors (3) beruht.

2. Prüfungsverfahren nach Anspruch 1, wobei die Schritte (a) bis (e) durch einen Computer ausgeführt werden, wobei der Computer zur Steuerung des Druckluftkompressors (3) und des Füllventils (5) und des Belüftungsventils (6) programmiert ist.

3. Medizinische Druckaufbau-Vorrichtung zur Anwendung an einem Patienten für die Unterstützung der Blutzirkulation im Körper, aufweisend einen Druckluftkompressor (3) und einen mit dem Druckluftkompressor (3) gekoppelten aufblasbaren Balg (2), ein Füllventil (5), welches den Lufteintritt in den Balg (2) ermöglicht und ein Entlüftungsventil (6) zur Entlüftung der Vorrichtung, wobei die Füll- und Entlüftungsventile (5, 6) selektiv bedienbar sind, um bei bestimmungsgemäßer Anwendung am Patienten regelmäßig zyklische Druckluft-Pulse vom Druckluftkompressor (3) zum Aufblasen des Balges (2) bereitzustellen, wobei die Vorrichtung **gekennzeichnet ist durch** eine Computer Steuerung, welche für eine der bestimmungsgemäßen Anwendung vorausgehende selektive Steuerung des Füllventils (5) und des Entlüftungsventils (6) programmiert ist, wobei die selektive Anwendung zur Leckprüfung an der Vorrichtung vorausgehend zum normalen Einsatz aufweist:
a. Öffnen des Füllventils (5) und des Entlüftungsventils (6) zur Entlüftung der Vorrichtung auf atmosphärischen Druck,
b. Schließen des Entlüftungsventils (6),
c. Beaufschlagen der Vorrichtung mit Druck mittels des Druckluftkompressors (3) bis zu einem bestimmten Schwellenwert,
d. Überwachen des Druckgradienten zum Schwellenwert über einen Zeitraum,
e. Vergleichen des Druckgradienten über den Zeitraum oder zu Zeitintervallen innerhalb des Zeitraumes mit einem vorher festgelegten Druckgradienten, welcher die Integrität der Vorrichtung bezogen auf die Undichtigkeit der Vorrichtung anzeigt, wobei zur Feststellung einer Undichtigkeit der Vorrichtung der vorher festgelegte Druckgradient auf dem Erreichen eines Druckes in der Vorrichtung von 10 mmHg innerhalb von 3 Sekunden nach Einschalten des Druckluftkompressors (3) beruht.

## Revendications

1. Un procédé de détection de présence de fuites dans un appareil médical pressurisé (1) utilisé sur des patients pour mettre en évidence la circulation sanguine de leur corps, procédé qui est mis en oeuvre avant l'utilisation normale de l'appareil sur le patient, et qui comprend un compresseur d'air (3) et une poche gonflable ou une vessie (2) couplés au compresseur d'air par l'intermédiaire d'une valve de remplissage (5) permettant à l'air de passer dans la vessie (2) et d'une valve d'évacuation (6) permettant à l'air de quitter l'appareil. La valve de remplissage (5) et la valve d'évacuation (6) fonctionnent de manière sélective pendant ladite utilisation normale de l'appareil en vue de délivrer des impulsions d'air à intervalles cycliques pour gonfler ou dégonfler la vessie (2). Le procédé de détection est **caractérisé en ce qu'**il comprend :
a, l'ouverture de la valve de remplissage (5) et de la valve d'évacuation (6) afin de soumettre l'appareil à la pression atmosphérique,
b. la fermeture de la valve d'évacuation (5),
c. la mise sous pression de l'appareil au moyen d'un compresseur d'air (3) jusqu'à une valeur plancher prédéterminée,
d. le contrôle, sur une certaine période de temps, du gradient de pression à ladite valeur plancher et
e. la comparaison du gradient de pression, sur ladite période de temps ou à intervalles de temps réguliers à l'intérieur de ladite période de temps, avec un gradient de pression prédéterminé, indicateur de l'intégrité du système en ce qui concerne une quelconque fuite dans l'appareil, comparaison par laquelle il est possible de déterminer toute présence ou l'absence de fuite d'air à l'intérieur de l'appareil, ledit gradient de pression prédéterminé étant calculé pour atteindre une pression dans l'appareil de 10mmHg dans
les trois secondes que prend la mise en marche dudit compresseur d'air.

2. Un procédé de détection selon la revendication 1 dans lequel les étapes (a) intermédiaires (e) sont réalisées par un ordinateur programmé pour actionner le compresseur d'air (3), la valve de remplissage (5) et la valve d'évacuation (6).

3. Un appareil de gonflage utilisé sur des patients pour mettre en évidence la circulation sanguine de leur corps, qui comprend un compresseur d'air (3) et une vessie gonflable (2) couplée au compresseur d'air (3), une valve de remplissage (5) permettant à l'air de passer dans la vessie (2), une valve d'évacuation (6) permettant à l'air de quitter l'appareil, les valves de remplissage et d'évacuation (5,6) fonctionnant de manière spécifique pour délivrer des impulsions d'air pressurisé en provenance du compresseur (3), afin de gonfler périodiquement la vessie (2), en utilisation normale sur le patient. L'appareil est **caractérisé en ce qu'**il est programmé informatiquement pour actionner spécifiquement la valve de remplissage (5) et la valve d'évacuation (6), avant utilisation normale de l'appareil, afin de détecter toute fuite d'air en son sein, avant utilisation normale de l'appareil, Cette mise en fonctionnement spécifique comprend :
a. l'ouverture de la valve de remplissage (5) et de la valve d'évacuation (6) afin d'appliquer une pression atmosphérique à l'appareil,
b. la fermeture de la valve (6),
c. la montée en pression de l'appareil au moyen d'un compresseur d'air (3) jusqu'à une valeur plancher prédéterminée,
d. le contrôle, sur une certaine période de temps, du gradient de pression à ladite valeur plancher
et
la comparaison du gradient de pression, sur ladite période de temps ou à intervalles de temps réguliers à l'intérieur de ladite période de temps, avec un gradient de pression prédéterminé, indicateur de l'intégrité du système en ce qui concerne une quelconque fuite dans l'appareil, comparaison par laquelle il est possible de déterminer toute présence de fuite d'air à l'intérieur de l'appareil, ledit gradient de pression prédéterminé étant calculé pour atteindre une pression dans l'appareil de 10mmHg dans les trois secondes que prend la mise en marche dudit compresseur d'air (3).
